# EUROPEAN PATENT APPLICATION

(11) **EP 1 987 763 A2**
(43) Date of publication of application: **05.11.2008**
(21) Application number: 08008202.7
(22) Date of filing: 29.04.2008
(51) Int. Cl.: A61B 5/00

(54) **System and method for measuring and displaying health information**

(30) Priority: 30.04.2007 US 797091
(71) Applicant: TANITA CORPORATION, Tokyo 174-8630 (JP)
(72) Inventor: Nakada, Masato, Itabashi-ku Tokyo 174-8630 (JP)
(74) Representative: Müller-Boré & Partner Patentanwälte

(57) **Abstract**

A system and method are provided for measuring and displaying health information. The system includes a measurement platform for performing one or more health information measurements, a terminal device for displaying the measurement results, and a portable storage device for storing the measurement information and user data. Push-pull output logic is used to provide for an efficient supply of power and to eliminate the consumption of power by the terminal device at the time of a signal input.

## Description

### Field of the Invention

This application relates generally to measuring health information, and more specifically to a system and method of communicating health information between a portable device and health measurement equipment.

### Background of the Invention

Many devices exist for acquiring and measuring user health information such as body weight, body fat percentage, pulse, heart rate, and/or other health information. For example, scales are commonly used to measure a user's weight and/or body mass. Exercise equipment, such as treadmills and stationary bicycles are often configured to measure the amount of calories a user has burned, or to measure a user's heart rate. Devices such as the aforementioned measuring devices are often configured to receive input from a user, such as the users age or sex.

When a user wishes to use a health measuring device, the user can input the information directly into the device. While some measurement devices such as a scale may be configured to store the user's information, many devices such as exercise equipment do not have this capability, especially those pieces of equipment used in a public place such as a gym or health club. Furthermore, these traditional measuring devices do not communicate with each other to share user information.

A portable storage device should be capable of remaining connected to a terminal device while minimizing current and power consumption. Prior art systems exist for inserting a portable device into an interface. However, these portable devices have an open-collector type output block which consumes large amounts of power and current.

It would be desirable to have a portable storage device for efficiently communicating with a terminal device, such as health measurement devices.

### Summary of the Invention

A system and method are provided for measuring and displaying health information. The system and method enables user information as well as data provided by a health measuring instrument to be exchanged among various health measuring instruments.

According to an embodiment, a system for measuring and displaying health information comprises a measurement platform configured to perform one or more health information measurements, a terminal device for displaying measurement results, and a portable storage device for storing the measurement information and other user data, wherein at least one of the terminal device and the portable storage device are configured using push-pull output logic.

The portable storage device may be configured to receive power from the terminal device. According to some embodiments, the portable storage device comprises a capacitor configured to store an electric charge. The portable storage device may be further configured to control the operating state of the terminal device.

The terminal device may comprise one or more health measurement instruments. The health measurement instruments may include one or more of a body composition analyzer, a pedometer, and a fitness machine.

According to some embodiments, the portable storage device may be inserted directly into the measurement platform ox may be indirectly connected to the measurement platform. The terminal device may use the measurement information and the user data stored on the portable storage device to compute one or more body composition items. The measurement platform and the terminal device may be integral.

According to some embodiments, a portable storage device far interfacing with health equipment comprises an active terminal configured to power the portable storage device and to input and output data to and from one or more external devices, a common terminal, and a storage module for storing data received from the one or more external devices, wherein the active terminal is configured using push-pull output logic.

The active terminal of the portable storage device may receive power from the one or more external devices. According to some embodiments, a capacitor may be included to store an electric charge enabling the portable storage device to remain operation when power is not supplied from the one or more external devices.

According to some embodiments, the portable storage device may be cylindrical in shape. The active terminal may comprise a first electrode exposed at a first end of the portable storage device and the common terminal may comprise a second electrode.

A method of exchanging data with a health measurement device which includes a measurement platform and a terminal device comprises receiving, at a portable storage device, an initialization command from the health measurement device, receiving a request from the health measurement device for user data, transmitting the requested user data to the health measurement device, an d receiving one or more body composition data items from the health measurement device, the one or more body composition data items computed by the health measurement device based on the user data, wherein the health measurement device supplies power to the portable storage device while exchanging data.

### Brief Description of the Drawings

Figure 1 depicts an overall system diagram, according to one embodiment.

Figure 2 depicts a block diagram of a terminal device, in accordance with some embodiments.

Figure 3 depicts a block diagram of a portable storage device, in accordance with some embodiments.

Figure 4 depicts a schematic diagram of a terminal device, in accordance with some embodiments.

Figure 5 depicts a schematic diagram of a portable storage device, in accordance with some embodiments.

Figure 6 depicts a portable storage device, in accordance with some embodiments.

Figure 7 is a flowchart depicting a method of exchanging data between a terminal device and a portable storage device, in accordance with some embodiments.

### Detailed Description

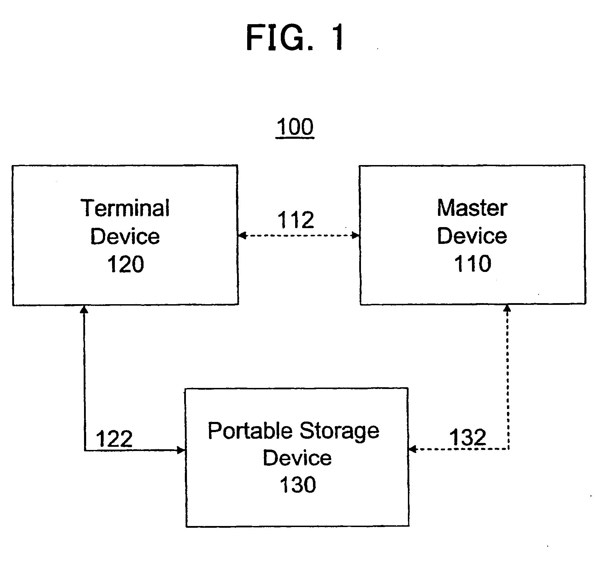

A system and method are described herein for interfacing a portable storage device and one or more pieces of health equipment. Figure 1 depicts an overall system diagram of a system 100 for using data stored on a portable storage device for measuring and displaying health information. System 100 may include measurement device 110, terminal device 120, and portable storage device 130.

Measurement device 110 may be any device capable of performing health measurements such as, for example, a scale, a treadmill, a stationary bicycle, a blood pressure analyzer (sphygmomanometer), a pedometer, a body composition analyzer, and/or other health measurement devices. Measurement device 110 may be communicatively coupled to terminal device 120, as depicted by communication link 112. Terminal device 120 may used as an interface between measurement device 110 and portable storage device 130. As depicted by communication link 122, portable storage device 130 may be connected directly to terminal device 120. Portable storage device 130 may also be configured for connection directly to measurement device 110, as depicted by communication link 132. While terminal device 120 and measurement device 110 are depicted as separate devices, these devices may be integrated to form a single device.

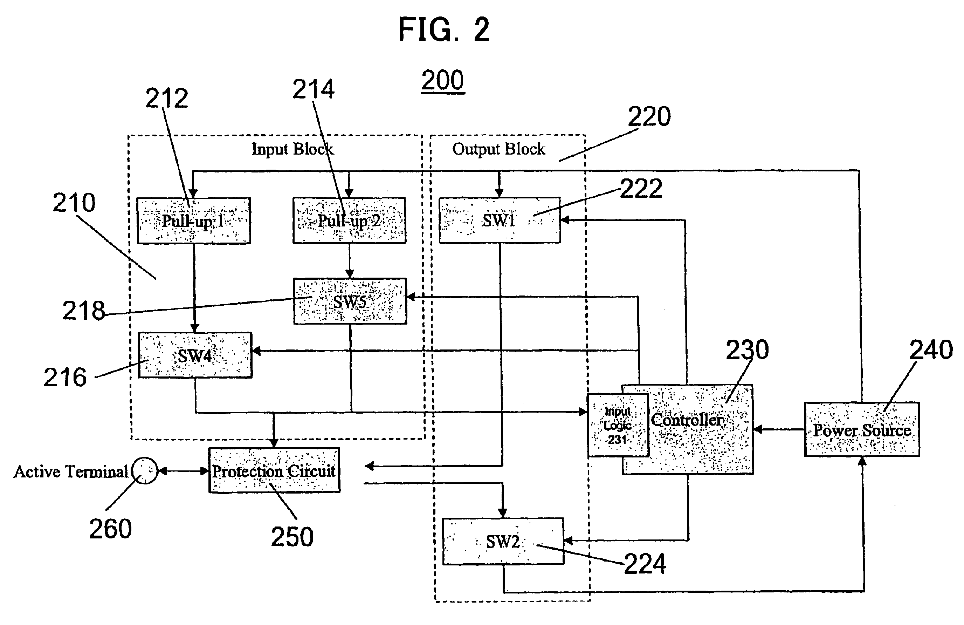

Figure 2 is a block diagram depicting terminal device 120 in greater detail. Terminal device 120 may include input block 210, output block 220, controller 230, power source 240, protection circuit 250, and active terminal 260. Active terminal 260 serves a connection point for connecting a portable storage device and may be configured to provide both signal and power to the portable storage device.

Input block 210 may be connected to an input logic 231 of controller 230 which is a high input impedance circuit. Input block 210 may comprise a first pull-up resistor 212, a second pull-up resistor 214, a first switching element 216, and a second switching element 218. First pull-up resistor 212 may be a weak pull-up resistor having a large value. First pull-up 212 resistor may be configured to detect when a portable storage device, such as portable storage device 110, is connected to terminal device 120. More particularly, first pull-up resistor 212 may be configured to limit current flow from terminal device 120 to a portable storage device when the terminal device has been connected, which indicates that a connection has occurred. Second pull-up resistor 214 may be configured to keep the voltage of the active terminal high even when no portable storage device is connected and the terminal device is accidentally changed to an input state. The value of second pull-up resistor 214 may be smaller than first pull-up resistor 212.

Switching elements 216 and 218 may be configured to select first pull-up resistor 212 or second pull-up resistor 214, respectively, under control of controller 230. Controller 230 may comprise a plurality of control lines used to control the operation of the terminal device, as described in detail below.

Terminal device 200 may be placed in an output mode when connected to either the high or low side of power source 240. Accordingly, output block 220 may include a first output switch 222 for connecting to the high side of the power source and second output switch 224 for connecting to the low side of the power source. Controller 230 may be configured to control the connection of the power source to the output block.

According to an exemplary embodiment, output block 220 operates using push-pull circuit logic which enables either terminal device 200 or a portable storage device to always be connected to either the high or low side of the power source at the time of output. This provides for an efficient supply of power and eliminates the consumption of power by the terminal device at the time of a signal input by disabling the output block 220.

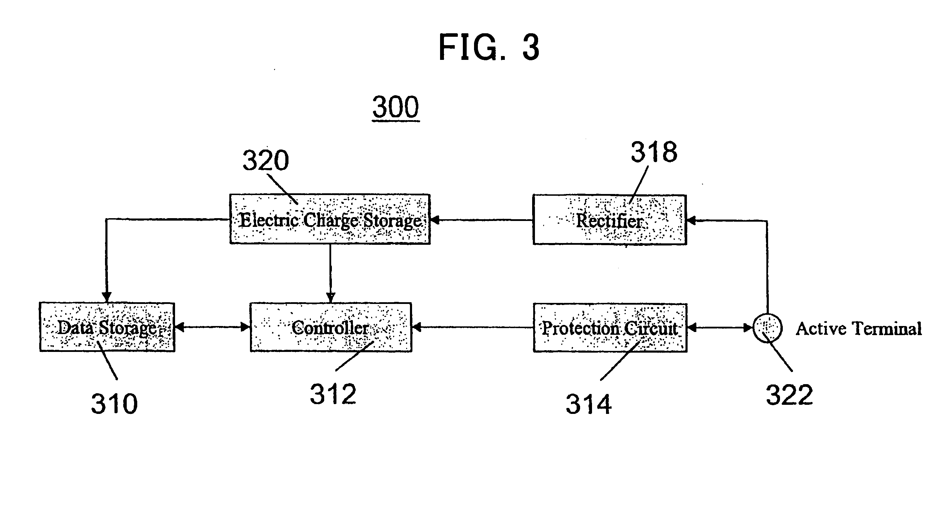

Figure 3 depicts a portable storage device 300 in accordance with some embodiments. Portable storage device 300 may include data storage mechanism 310, controller 312, protection circuit 314, rectifier 318, electric charge storage 320, and active terminal 322. While not depicted, portable storage device 300 also includes a ground terminal. Active terminal 322 acts as both a signal terminal and a power terminal.

Data storage mechanism 310 may include any rewriteable electronic memory such as, for example, flash ROM or EEPROM. Data storage mechanism 310 may be configured to store user data such, for example, an identification associated with the user, the user's age, date of birth, sex, and/or other user information. Additionally, data storage mechanism 3I0 may also store information such as measurements obtained from health measurement equipment, information related to the measurements such as the date and time the measurement was taken, settings associated with the equipment and/or the measurements, or information describing the measurement equipment such as the name of the equipment.

Controller 312 may be configured to direct the exchange of data between portable storage device 300 and one or more terminal devices. Portable storage device 300 may be configured to obtain power from a terminal device when connected to the terminal device. Electric charge storage unit 320 is configured to store a charge from an external power source, such as a power source of a connected terminal device. According to some embodiments, electric storage unit 320 comprises a capacitor configured to provide power to portable storage device when not receiving power from the terminal device.

Rectifier 318 may be configured to separate power and data signals. Since the voltage of the active terminal is almost directly connected with the input pin D1, power may be supplied via rectifier 318 when the voltage of the active terminal is higher than the voltage of the electric charge storage 320. Accordingly, the power of portable storage device 300 may be sourced by a terminal device when the terminal device is in an output state and the high side of the signal is output. If either the terminal device or portable storage device 300 outputs the low side signal and the active terminal goes low, current may not flow reversely by the rectifier 318, and the electric charge in the electric charge storage 320 remain.

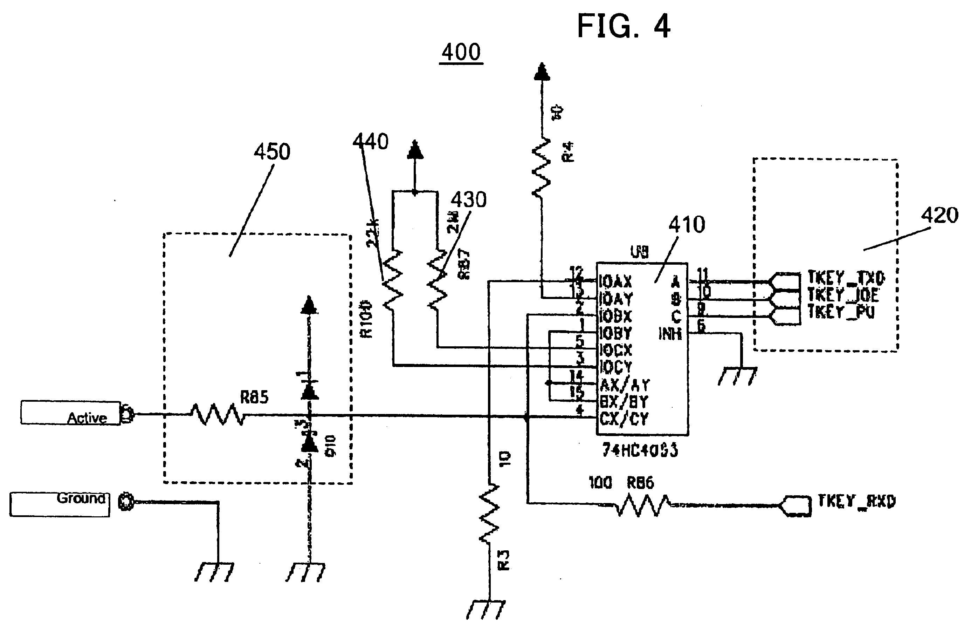

Figure 4 is an example of a terminal device circuit 400 which may be used to implement the terminal device depicted in Figure 2. Circuit 400 may include a controller 410, a first pull-up resistor 430, a second pull-up resistor 440, and a protection circuit 450. In Figure 4, controller 410 is depicted as a. multiplexer integrated circuit. Accordingly, controller 410 performs the s bewitching operations of the input and output switches depicted in Figure 2. However, other control device may be used such as, for example, transistors, MOSFETS, digital switches, and/or other control devices.

Controller 410 controls the operation of terminal device 400 using a plurality of control lines 420. Control lines 420 may include a transmission control line TKEY_TXD, an input/output control line TKEY_IOE, a pull-up control line TKEY_PU, and a reception control line TKEY_RXD. TKEY_IOE may be used to select either an output or an input mode for terminal device 400. TKEY_PU may be configured to select either the first pull-up resistor 420 or the second pull-up resistor 440.

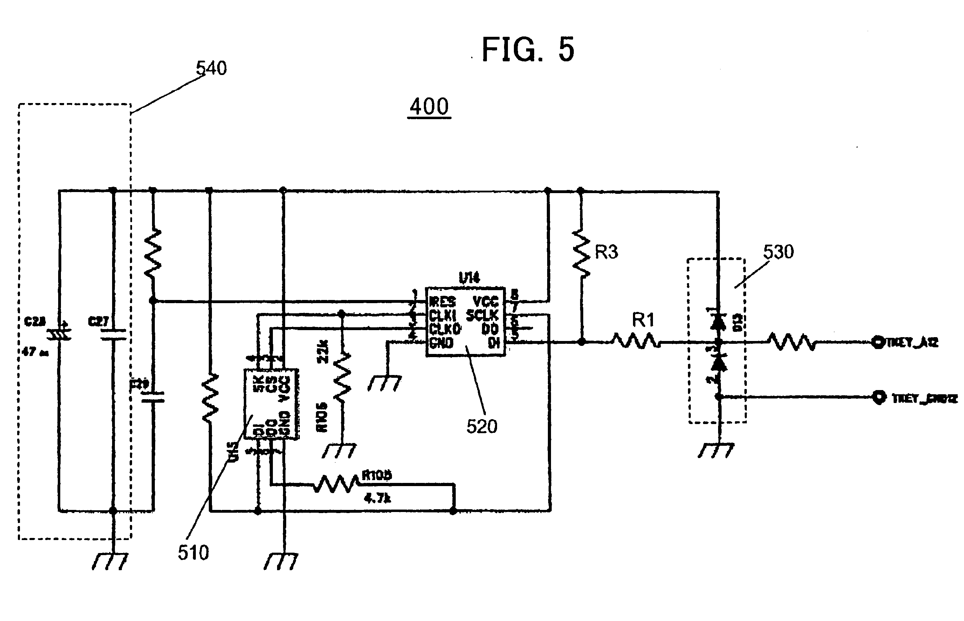

Figure 5 is an example of a circuit which may be used to implement the portable storage device depicted in Figure 3. Portable storage device circuit 500 may comprise memory device 510, microcontroller 520, rectifier 530, and power storage capacitors 540. Controller 520 comprises a pin D1 which connects to the active terminal of the portable storage device via resistors R1 and R2 which form a protection circuit. Resistor R3 may act as a weak pull-up resistor when receiving a signal. Pin D1 is used to control data input and output. In the output mode, D1 is configured using push-pull logic. As described above, push-pull logic enables either the portable storage device or a terminal device to always be connected to either the high or low side of the power supply.

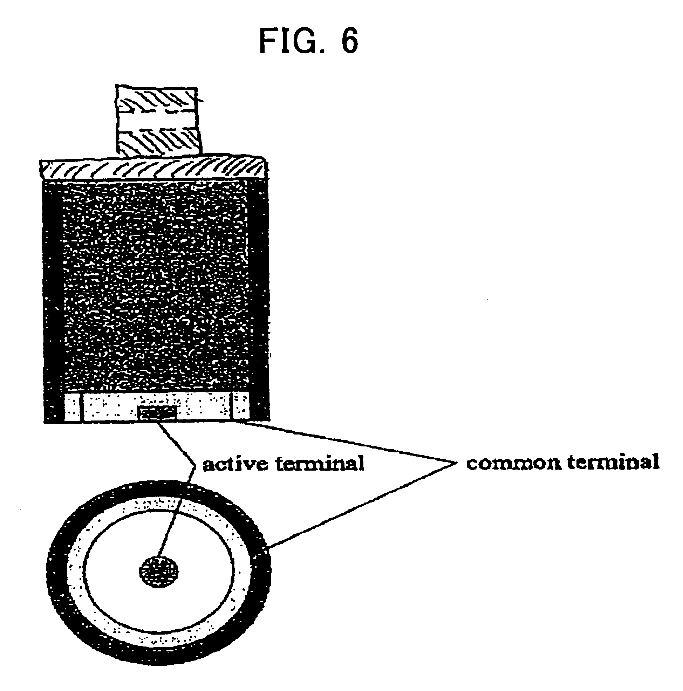

Figure 6 is one example of an external view of the portable storage device described herein. As shown in Figure 6, the portable storage device may be cylindrical in shape. The device includes an active terminal exposed at one end which connects to a terminal device and a common terminal which serves as a ground terminal. Other external configurations may also be used. For example, the common terminal may either be isolated to the terminal to the cylinder or attached to the cylinder. The portable storage device may include a protrusion at the opposite end of the device having a through hole therein. This protrusion may be used for connecting the portable device to a key ring or the like.

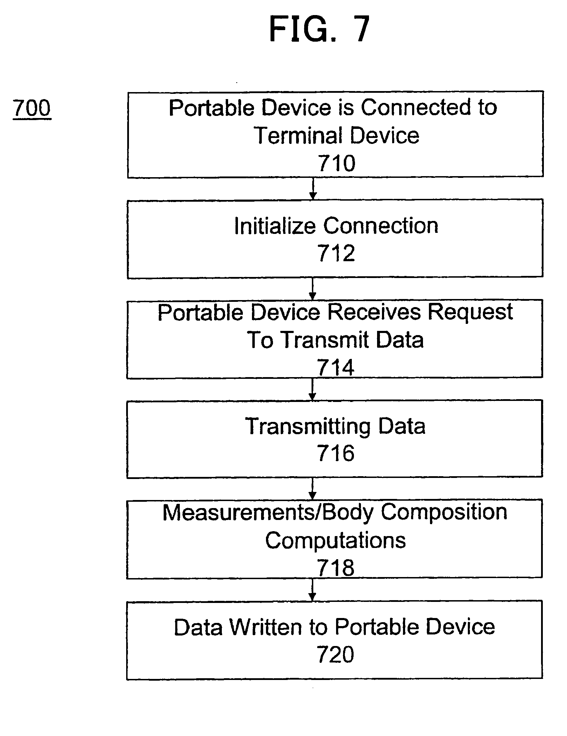

Figure 7 is a flowchart depicting a method 700 of exchanging data between a health device and portable storage device. As depicted at 710, the process may begin when a portable storage device is inserted into a health measurement device. In accordance with some embodiments of the invention, health measurement devices and the portable storage device may be configured to remain in a standby mode until being activated. The health measurement device may recognize that a portable storage device has been connected by detecting the active terminal transistor from high to low by a weak pull-up resistor and input logic. Additionally, the portable storage device may be configured to issue an activation request upon connection to the health measurement device, and may cause the health measurement device to return to the standby mode once the connection has been removed.

As depicted at 712, upon activation of the health measurement device, the portable storage device may receive an initialization command from the health measurement device. The health measurement device may transition from the standby state to an output state in order to issue an initialization command to the portable storage device. Initialization may include transmitting a predetermined code. If the portable storage device receives a code other than the predetermined code, this may indicate an error has occurred, and another initialization command may be issued. According to some embodiments, if initialization fails after a predetermined number of attempts, an error message may be displayed to the user.

Upon initialization, the health measurement device may then issue a command to the portable storage device to retrieve user data, as depicted at 714. The terminal device will not continue to supply power to the portable storage device while receiving the requested user data but operates with its own electric charges stored by an electric charge storage device. The portable device may respond by transmitting the requested user data, as depicted at 716. The response may include, for example, a header followed by one or more data items including data length, user identification, user gender, date of birth, height, life intensity, user name, and are other data.

As depicted at 718, the terminal device may calculate one or more body composition items based on the retrieved user data. Different terminal devices may use different data in performing calculations. For example, a body mass measuring device may use data such as the user's age, gender, height, and activity level coupled with a measurement of weight and impedance from a measuring platform to calculate a user's body composition. Exercise equipment, such as a treadmill or stationary bicycle may use user data, such as a previously calculated and stored weight or muscles mass to more precisely calculate a user's calorie consumption. The terminal device may be configured to store the calculated body composition items, as depicted at 720.

The previous description of the disclosed embodiments is provided to enable any person skilled in the art to make or use the described embodiments. Various modifications to these embodiments will be readily apparent to those skilled in the art, and the generic principles defined herein may be applied to other embodiments without departing from the spirit or scope. Thus, the disclosure is not intended to be limited to the embodiments shown herein, but is to be accorded the full scope consistent with the claims.

## Claims

1. A system for measuring and displaying health information, comprising: a measurement platform configured to perform one or more health information measurements; a terminal device for displaying measurement results; and a portable storage device for storing the measurement information and user data, wherein at least one of the terminal device and the portable storage device are configured using push-pull output logic.

2. The system of claim 1, wherein the portable storage device receives power from the terminal device, and
wherein the portable storage device preferably comprises: a capacitor configured to store an electric charge.

3. The system of claim 1 or 2, wherein the terminal device comprises one or more health measurement devices, and
wherein the health measurement devices preferably include one or more of a body composition analyzer, a pedometer, and a fitness machine.

4. The system of any of claims 1 to 3, wherein the portable storage device is further configured to control the operating state of the terminal device.

5. The system of any of claims 1 to 4, wherein the portable storage device is inserted directly into the measurement platform, or
wherein the portable storage device is indirectly connected to the measurement platform.

6. The system of any of claims 1 to 5, wherein the terminal device uses the measurement information and user data stored on the portable storage device to compute one or more body composition items.

7. The system of any of claims 1 to 6, wherein the measurement platform and the terminal device are integral.

8. A portable storage device for interfacing with health equipment, the portable storage device comprising: at least one active terminal configured to power the portable storage device and to input and output data to and from one or more external devices; a common terminal; and a storage module for storing data received from the one or more external devices, wherein the active terminal in configured using push-pull output logic.

9. The portable storage device of claim 8, wherein the at least one active terminal receives power from the one or more external devices.

10. The portable storage device of claim 8 or 9, further comprising: a capacitor configured to store an electric charge enabling the storage device to remain operational when power is not supplied from the one or more external devices.

11. The portable storage device of any of claims 8 to 10, wherein the one or more external devices comprise one or more of a body composition analyzer, an activity monitor, a fitness machine, and a personal computer.

12. The portable storage device of any of claims 8 to 11, wherein the storage module stores user information entered by the user into the external device.

13. The portable storage device of any of claims 8 to 12, wherein: the at least one active terminal comprises a first electrode exposed at a first end of the storage device.

14. The portable storage device of any of claims 8 to 13, wherein the storage device is cylindrical in shape, and the ground terminal comprises a second terminal electrode.

15. The portable storage device of any of claims 8 to 14, further comprising: a protrusion provided at a second end of the storage device having a through hole therein.

16. A method of exchanging data with a health measurement device which includes a measurement platform and a terminal device, the method comprising: receiving, at a portable storage device, an initialization command from the health measurement device; upon initialization, receiving a request from the health measurement device for user data; transmitting the requested user data to the health measurement device; and receiving one or more body composition data items from the health measurement device, the one or more body composition data items computed by the health measurement device based on the user data, wherein the health measurement device supplies power to the portable storage device while exchanging data.

17. The method of claim 16, wherein transmitting the requested user data requires transmitting a header and one or more of a user identification code, user gender, user data of birth, user height, and user name.

18. The method of claim 16 or 17, wherein the portable storage device is configured to activate and deactivate the health measurement device, and the initialization command is received after the health measurement device has been activated.

19. The method of any of claims 16 to 18, wherein the portable storage device and the health measurement device output data using push-pull logic.
